# EUROPEAN PATENT APPLICATION

(11) **EP 3 964 222 A1**
(43) Date of publication of application: **09.03.2022**
(21) Application number: 20798407.1
(22) Date of filing: 24.04.2020
(51) Int. Cl.: A61K 36/718, A61K 36/538, A61P 29/00, A61P 1/00, A23L 33/105, A23K 10/30

(54) **PHARMACEUTICAL COMPOSITION COMPRISING MIXTURE EXTRACT OF COPTIS DELTOIDEA AND SCHIZONEPETA TENUIFOLIA AS ACTIVE INGREDIENT FOR PREVENTION OR TREATMENT OF INFLAMMATORY BOWEL DISEASE**

(30) Priority: 29.04.2019 KR 20190050088
(71) Applicant: Helixmith Co., Ltd., Gangseo-gu Seoul 07794 (KR)
(72) Inventor: LEE, Wonwoo, Seoul 05587 (KR); LEE, Doo Suk, Anyang-si Gyeonggi-do 14069 (KR)
(74) Representative: J A Kemp LLP
(86) International application number: PCT/KR2020/005475
(87) International publication number: WO 2020/222470

(57) **Abstract**

The present invention relates to a pharmaceutical composition comprising a mixture extract of Coptis deltoidea and Schizonepeta tenuifolia as an active ingredient for prevention or treatment of an inflammatory bowel disease. The use of Coptis deltoidea and Schizonepeta tenuifolia extracts of the present invention can provide a pharmaceutical composition for prevention or treatment of an inflammatory bowel disease, the pharmaceutical composition being capable of being used safely on the human body without toxicity and side effects, or can provide a food composition and a feed composition each for prevention or alleviation of an inflammatory bowel disease.

## Description

### Technical Field

The present disclosure relates to a pharmaceutical composition comprising a mixed extract of Coptis Rhizome and *Schizonepeta tenuifolia* Briquet as an active ingredient for prevention or treatment of inflammatory bowel disease.

This application claims priority to and the benefit of Korean Patent Application No. 10-2019-0050088 filed in the Korean Intellectual Property Office on 29 April 2019, the disclosure of which is incorporated herein by reference.

### Background Art

Inflammatory disease is one of the most important health problems all over the world. Inflammation is generally a protective response of body tissues to foreign substances or injurious stimuli. The causes of inflammation are associated with: infectious causes, such as bacteria, viruses, and parasites; physical causes, such as burns or radiation; chemicals, such as toxins, drugs, or industrial agents; immune responses, such as allergic and autoimmune responses, or oxidative stress.

Inflammation is characterized by pain, redness, swelling, fever, and loss of function of the affected area. These symptoms are the results of complex interactions that take place between the cells of the immune system. The response of the cells results in an interacting network of several inflammatory mediators.

On the other hand, one of the representative inflammatory diseases is inflammatory bowel disease (IBD). Inflammatory bowel disease refers to a chronic inflammatory disease in which inflammation or ulceration occurs in the intestine due to unknown causes, for example, ulcerative colitis (UC), Crohn's disease (CD), and Behcet's disease.

Inflammatory bowel disease is known to exhibit intestinal pathological signs, such as weight loss, diarrhea accompanied by mucus or blood, fever, intestinal motility disorder, and shorting of colon. Almost all patients with ulcerative colitis have inflammation in the rectum, and the inflammation occurs throughout the large intestine. It is therefore known that in inflammatory bowel disease, various inflammatory cytokines are secreted from the intestinal mucosa and inflammation signaling is activated.

Ulcerative colitis is a relapsed-refractory disease of the bowel in the digestive system, and in clinical practice, main symptoms thereof are abdominal pain, diarrhea, and bloody mucus excrement, and lesions are mainly found in the mucosa and submucosa. Normal colon tissue is divided into four layers: mucosa, submucosa, muscle layer and serous membrane. The development of ulcerative colitis causes pathological changes in the mucosa and submucosa. As the ulcer is formed, inflammation occurs in adjacent layers, into which cells then infiltrate, and non-specific changes, for example, the generation of crypt abscess, the occurrence of inflammation in surrounding blood vessels, and the appearance of various types of inflammatory cells, such as neutrophils and eosinophils, are observed. This disease is common in countries that mainly eat meat, such as European countries and the United States, and has recently been significantly increasing in Korea. However, the causes and pathogenesis of this disease are comparatively complex, and thus are still unknown.

Crohn's disease is chronic inflammatory bowel disease, which is one of the autoimmune diseases, and most cases are known to occur at the border between the small and large intestine. This disease was discovered by the American doctor Crohn in 1932 and named Crohn's disease. Crohn's disease is a refractory disease, and the causes of the disease have not yet been clearly established since the disease was discovered not long ago. The majority opinion is that this disease is caused by an excessive immune response in the immune system, but not a certainty. There is no cure for this disease, and factors, such as eating habits, intestinal infection, and antibiotic utilization, are likely to have an influence. In Crohn's disease, inflammation is found sporadically in the gastrointestinal tract and affects transmural layers of the intestine. This may cause the formation of fistulas in the intestine, causing a complication in which the intestine adheres to other organs.

As treatments for inflammatory bowel disease, 5-aminosalicylic acid (5-ASA)-based drugs that block the production of prostaglandins, for example, sulfasalazine, or immunosuppressants, such as steroids, are currently used. However, these drugs, when taken for a long time, may cause side effects, toxicity, and tolerance, with respect to dosages thereof.

Meanwhile, natural product extracts, or compositions including the same have been used to alleviate and treat various diseases including inflammation. The reason is that natural products are extremely diverse and have high specific physiological activity and have advantages of significantly reducing the side effects of chemical drugs. Therefore, there is a need for developing effective treatment methods or drugs for inflammatory bowel disease using these natural products.

### Disclosure of Invention

### Technical Problem

The present inventors conducted intensive research efforts to develop natural product medicines effective in inflammatory bowel disease. As a result, the present inventors established that a mixed extract of Coptis Rhizome and *Schizonepeta tenuifolia* Briquet has an excellent anti-inflammatory effect, and thus completed the present disclosure.

Accordingly, an aspect of the present disclosure is to provide a pharmaceutical composition comprising a mixed extract of Coptis Rhizome and *Schizonepeta tenuifolia* Briquet as an active ingredient for preventing or treating inflammatory bowel disease.

Another aspect of the present disclosure is to provide a food composition comprising a mixed extract of Coptis Rhizome and *Schizonepeta tenuifolia* Briquet as an active ingredient for preventing or alleviating inflammatory bowel disease.

Still another aspect of the present disclosure is to provide a feed composition comprising a mixed extract of Coptis Rhizome and *Schizonepeta tenuifolia* Briquet as an active ingredient for preventing or alleviating inflammatory bowel disease.

Still another aspect of the present disclosure is to provide a method for preventing, alleviating, or treating inflammatory bowel disease, the method including administering a subject a pharmaceutical composition or food composition comprising a mixed extract of Coptis Rhizome and *Schizonepeta tenuifolia* Briquet as an active ingredient.

### Solution to Problem

In accordance with an aspect of the present disclosure, there is provided a pharmaceutical composition comprising a mixed extract of Coptis Rhizome and *Schizonepeta tenuifolia* Briquet as an active ingredient for prevention or treatment of an inflammatory bowel disease.

As used herein, the term "mixed extract" or "mixed herbal extract" encompasses all of a mixture of a Coptis Rhizome and a *Schizonepeta tenuifolia* Briquet extract or an extract of a mixture of Coptis Rhizome and *Schizonepeta tenuifolia* Briquet.

Coptis Rhizome is a medicinal herb of herbaceous perennial plant of the *Ranunculaceae* family, and refers to the rhizome of *Coptis japonica* Makino, *Coptis chinensis* Franchet, *Coptis deltoidea* CY Cheng et Hsiao, or *Coptis teeta* Wallich, with roots removed.

*Schizonepeta tenuifolia* Briquet is an annual plant belonging to the *Labiatae* family, and refers to *Schizonepetae Herba* as the above-ground part thereof or *Schizonepetae Spica* as the flower stalk thereof.

As used herein, the term "inflammatory bowel disease" refers to a disease in which inflammation occurs in the intestine, that is, small intestine and large intestine, and the inflammatory bowel disease includes a disease in which abnormal chronic inflammation in the intestine repeat remission and recurrence. The inflammatory bowel disease includes specific enteritis with known causes, non-specific enteritis with unknown causes, and enteritis caused from other diseases, for example, intestinal Behcet's disease.

As used herein, the term "treatment" refers to any action that attain the remission or complete recovery of the symptoms of inflammatory bowel disease through the administration of the composition according to the present disclosure.

As used herein, the term "prevention" refers to any action that inhibit or delay the symptoms of inflammatory bowel disease through the administration of the composition according to the present disclosure.

As used herein, the term "comprising as an active ingredient" refers to the inclusion of an amount that is sufficient to attain efficacy or activity of the mixed extract of Coptis Rhizome and *Schizonepeta tenuifolia* Briquet.

The mixed extract of Coptis Rhizome and *Schizonepeta tenuifolia* Briquet, contained in the composition of the present disclosure, is a natural plant material and has no cytotoxicity. The quantitative upper limit of the mixed extract of Coptis Rhizome and *Schizonepeta tenuifolia* Briquet, contained in the composition of the present disclosure, may be selected within an appropriate range by a person skilled in the art.

As used herein, the term "extract" has a meaning that is commonly used as a crude extract in the art, but broadly, encompasses a fraction obtained by additionally fractionating the extract. That is, the extract of Coptis Rhizome and *Schizonepeta tenuifolia* Briquet includes not only one obtained by using an extraction solvent but also one obtained by additionally applying a purification procedure to the same. For example, fractions obtained through various purification methods that are additionally conducted, such as a fraction obtained by passing the extract through an ultrafiltration membrane with a predetermined molecular weight cut-off value and a fraction obtained by various types of chromatography (fabricated for separation depending on size, charge, hydrophobicity, or hydrophilicity), are also included in the extract of the Coptis Rhizome and *Schizonepeta tenuifolia* Briquet of the present disclosure.

The pharmaceutical composition of the present disclosure may comprise a pharmaceutically acceptable carrier. The pharmaceutically acceptable carrier is typically used at the time of formulation, and examples thereof may include lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oils, and the like, but are not limited thereto. The pharmaceutical composition of the present disclosure may further comprise, in addition to the above ingredients, a lubricant, a wetting agent, a sweetening agent, a flavoring agent, an emulsifier, a suspending agent, a preservative, and the like. Suitable pharmaceutically acceptable carriers and preparations are described in detail in Remington's Pharmaceutical Sciences (19th ed., 1995) .

In addition, the pharmaceutical composition of the present disclosure may further comprise an active ingredient that is known to have a treatment effect on inflammatory bowel disease in the art. Examples thereof include steroids, such as glucocorticosteroid, 5-aminosalicylic acid (5-ASA)-based drugs, such as sulfasalazine and mesalazine, anti-TNF-α monoclonal antibodies, and the like.

The pharmaceutical composition of the present disclosure may be administered orally or parenterally, and examples of parenteral administration may include intravenous injection, subcutaneous injection, intramuscular injection, intraperitoneal injection, percutaneous administration, and the like.

The appropriate dose of the pharmaceutical composition of the present disclosure varies depending on factors, such as a formulating method, a manner of administration, patient's age, body weight, sex, and morbidity, a food, a time of administration, a route of administration, an excretion rate, and response sensitivity. The ordinarily skilled practitioner can easily determine and prescribe a dose that is effective for the desired treatment or prevention. According to a preferable embodiment of the present disclosure, the daily dose of the pharmaceutical composition of the present disclosure is 0.001-1000 mg/kg.

The pharmaceutical composition of the present disclosure may be prepared into a unit dosage form or in the form of being contained in a multi-dose container by using a pharmaceutically acceptable carrier and/or excipient according to a method that can be easily performed by a person skilled in the art to which the present disclosure pertains. The formulation may be in the form of a solution in an oily or aqueous medium, a suspension, or an emulsion, or in the form of an extract, a powder, granules, a tablet, or a capsule, and may further contain a dispersant or a stabilizer.

In an embodiment of the present disclosure, the inflammatory bowel disease is selected from the group consisting of ulcerative colitis, Crohn's disease, intestinal Behcet's disease, indeterminate colitis, bacterial enteritis, viral enteritis, amoebic enteritis, hemorrhagic rectal ulcer, ischemic colitis, and tuberculous enteritis, but is not limited thereto. More specifically, the inflammatory bow disease is ulcerative colitis or Crohn's disease.

In an embodiment of the present disclosure, the pharmaceutical composition inhibits the activation of TNF-α, IL-1β, IL-17, IL-23, CCL2, or MIP-2, which is an inflammatory factor.

As used herein, the term "activation" refers to the activation of a corresponding gene or the production of a functional protein of a corresponding gene by expression. The meaning of "inhibiting activation" is that a corresponding gene is not expressed, or a functional protein of a corresponding gene is not produced even if the corresponding gene is expressed, or includes a case in which there is no substantial expression due to a significant low level of expression.

In an embodiment of the present disclosure, the mixed extract of Coptis Rhizome and *Schizonepeta tenuifolia* Briquet is obtained by mixing Coptis Rhizome and *Schizonepeta tenuifolia* Briquet at a weight ratio of 1-30:1-30, 1-20:1-20, 1-15:1-15, 1-10:1-10, 1-8:1-8, 1-7:1-7, 1-6:1-6, 1-5:1-5, 1-4:1-4, 1-3:1-3, 1-2:1-2, or 1:1, but is not limited thereto. More specifically, the mixing weight ratio of Coptis Rhizome and *Schizonepeta tenuifolia* Briquet is 1-30:1, 1-20:1, 1-15:1, 1-10:1, 1-8:1, 1-7:1, 1-6:1, 1-5:1, 1-4:1, 1-3:1, 1-2:1, 20:1, 15:1, 10:1, 8:1, 7:1, 6:1, 5:1, 4:1, 3:1, 2:1, or 1:1.

According to an exemplary embodiment of the present disclosure, the mixed extract of Coptis Rhizome and *Schizonepeta tenuifolia* Briquet showed an excellent effect in the relief of inflammation when comprising Coptis Rhizome and *Schizonepeta tenuifolia* Briquet at a weight ratio of 1 to 10:1.

When the mixed extract of Coptis Rhizome and *Schizonepeta tenuifolia* Briquet, used in the composition of the present disclosure, is obtained by subjecting Coptis Rhizome, *Schizonepeta tenuifolia* Briquet, or a combination thereof to extraction with an extraction solvent, various extraction solvents may be employed. Preferably, a polar solvent or a non-polar solvent may be used. Appropriate examples of the polar solvent may include (i) water, (ii) an alcohol (preferably, methanol, ethanol, propanol, butanol, n-propanol, iso-propanol, or n-butanol), (iii) acetic acid, (iv) dimethyl-formamide (DMFO), and (v) dimethyl sulfoxide (DMSO).

Appropriate examples of the non-polar solvent include acetone, acetonitrile, ethyl acetate, methyl acetate, fluoroalkanes, pentane, hexane, 2,2,4-trimethyl pentane, decane, cyclohexane, cyclopentane, diisobutylene, 1-pentene, 1-chlorobutane, 1-chloropentane, o-xylene, diisopropyl ether, 2-chloropropane, toluene, 1-chloropropane, chlorobenzene, benzene, diethyl ether, diethyl sulfide, chloroform, dichloromethane, 1,2- dichloroethane, aniline, diethyl amine, ethers, carbon tetrachloride, and THF.

In the present disclosure, when Coptis Rhizome, *Schizonepeta tenuifolia* Briquet, or a combination thereof is subjected to extraction, hot-water extraction, cold extraction, reflux extraction, room-temperature extraction, or ultrasonic extraction may be used.

In an embodiment of the present disclosure, the mixed extract of Coptis Rhizome and *Schizonepeta tenuifolia* Briquet is obtained by extraction with at least one solvent selected from water, an alcohol having 1 to 4 carbon atoms, or a mixture thereof.

In an embodiment of the present disclosure, the alcohol is methanol, ethanol, propanol, butanol, n-propanol, iso-propanol, n-butanol. More specifically, the alcohol is ethanol.

In an embodiment of the present disclosure, the concentration of alcohol is 20 to 99 vol%. More specifically, the concentration of ethanol is 20 to 75 vol%, 20 to 55 vol%, 20 to 35 vol%, 25 to 99 vol% 25 to 75 vol%, 25 to 55 vol%, 25 to 35 vol%, 55 to 99 vol%, or 55 to 75 vol%, but is not limited thereto. Most preferably, the concentration of ethanol is 20 to 30 vol% or 65 to 75 vol%.

In an embodiment of the present disclosure, the mixed extract of Coptis Rhizome and *Schizonepeta tenuifolia* Briquet is a hot-water extract obtained by hot-water extraction at 70 to 100°C. More specifically, the mixed extract of Coptis Rhizome and *Schizonepeta tenuifolia* Briquet is a hot-water extract obtained by hot-water extraction at 75 to 100°C, 80 to 100°C, 85 to 100°C, 90 to 100°C, 75 to 95°C, 80 to 95°C, or 85 to 95°C, but is not limited thereto. Most specifically, the mixed extract of Coptis Rhizome and *Schizonepeta tenuifolia* Briquet is a hot-water extract obtained by hot-water extraction at 90 to 95°C.

According to an exemplary embodiment of the present disclosure, the mixed extract of Coptis Rhizome and *Schizonepeta tenuifolia* Briquet of the present disclosure showed a significant anti-inflammatory effect for both of a hot-water extract and ethanol extraction. Coptis Rhizome and *Schizonepeta tenuifolia* Briquet showed a significant anti-inflammatory effect when mixed at a weight ratio of 1:1, 2:1, 4:1, 8:1, or 10:1, and especially, showed an excellent anti-inflammatory effect when mixed at a weight ratio of 10:1.

In accordance with another aspect of the present disclosure, there is provided a food composition comprising a mixed extract of Coptis Rhizome and *Schizonepeta tenuifolia* Briquet for preventing or alleviating inflammatory bowel disease.

The food composition of the present disclosure may be prepared in the form of a powder, granules, a tablet, a capsule, a drink, or the like. Examples thereof are various foods such as candies, drinks, chewing gums, teas, vitamin complexes, health supplement foods, and the like.

The food composition of the present disclosure may comprise not only the mixed extract of Coptis Rhizome and *Schizonepeta tenuifolia* Briquet as an active ingredient, but also the ingredients that are typically added at the time of food manufacturing, for example, a protein, a carbohydrate, a fat, a nutrient, seasoning, and a flavoring agent. Examples of the carbohydrates are: saccharides, such as monosaccharides (e.g., glucose and fructose), disaccharides (e.g., maltose, sucrose, and oligosaccharides), and polysaccharides (e.g., dextrin and cyclodextrin); and sugar alcohols, such as xylitol, sorbitol, and erythritol. As the flavoring agent, natural flavoring agents (thaumatin, stevia extracts (e.g., rebaudioside A, glycyrrhizin, etc.)) and synthetic flavoring agents (saccharin, aspartame, etc.) may be used. For example, the food composition of the present disclosure, when prepared as a drink, may further comprise citric acid, liquefied fructose, sugar, glucose, acetic acid, malic acid, fruit juice, an Eucommia ulmoides extract, a jujube extract, a licorice extract, and the like, in addition to the mixed extract of Coptis Rhizome and *Schizonepeta tenuifolia* Briquet of the present disclosure.

In accordance with still another aspect of the present disclosure, there is provided a feed composition comprising a mixed extract of Coptis Rhizome and *Schizonepeta tenuifolia* Briquet for preventing or alleviating inflammatory bowel disease.

The mixed extract of Coptis Rhizome and *Schizonepeta tenuifolia* Briquet, contained in the composition of the disclosure, is a natural plant material, and Coptis Rhizome and *Schizonepeta tenuifolia* Briquet have long been used for an edible purpose and as folk medicines, and thus the extract of the present disclosure extracted therefrom can also be expected to have no toxicity and side effects. Therefore, the extract of Coptis Rhizome and *Schizonepeta tenuifolia* Briquet may be used as a medicinal, food, or feed composition.

Since the features of the extraction of Coptis Rhizome and *Schizonepeta tenuifolia* Briquet, contained in the food composition and the feed composition of the present disclosure, overlap with those of the extraction for the mixed extract of Coptis Rhizome and *Schizonepeta tenuifolia* Briquet, contained in the pharmaceutical composition, the overlapping description therebetween is omitted in order to avoid excessive complexity of the present specification.

In accordance with still another aspect of the present disclosure, there is provided a method for preparing a mixed extract of Coptis Rhizome and *Schizonepeta tenuifolia* Briquet, the method comprising the steps of: (i) extracting by applying an extraction solvent to Coptis Rhizome and *Schizonepeta tenuifolia* Briquet separately, or the mixed Coptis Rhizome and *Schizonepeta tenuifolia* Briquet; (ii) filtering the extracts, formed through the extraction step by stirring at room temperature, respectively; and (iii) concentrating the filtrate under reduced pressure.

In an embodiment of the present disclosure, the extraction solvent is methanol, ethanol, propanol, butanol, n-propanol, iso-propanol, n-butanol. More specifically, the alcohol is ethanol.

In an embodiment of the present disclosure, the concentration of alcohol is 20 to 99 vol%. More specifically, the concentration of ethanol is 20 to 75 vol%, 20 to 55 vol%, 20 to 35 vol%, 25 to 99 vol% 25 to 75 vol%, 25 to 55 vol%, 25 to 35 vol%, 45 to 99 vol%, or 45 to 75 vol%, but is not limited thereto. Most specifically, the concentration of ethanol is 45 to 70 vol%. According to an exemplary embodiment of the present disclosure, an excellent extraction yield was showed when the concentration of ethanol was 45 to 70 vol%.

Since the features of the extraction in the preparation method of the present disclosure overlap with those of the extraction for the mixed extract of Coptis Rhizome and *Schizonepeta tenuifolia* Briquet, contained in the pharmaceutical composition, the overlapping description therebetween is omitted in order to avoid excessive complexity of the present specification.

In accordance with still another aspect of the present disclosure, there is provided a method for preventing, alleviating, or treating inflammatory bowel disease, the method comprising administering a subject a pharmaceutical composition or food composition comprising the above-described mixed extract of Coptis Rhizome and *Schizonepeta tenuifolia* Briquet of the present disclosure as an active ingredient.

As used herein, the term "administration" or "administer" refers to the direct administration of a therapeutically or prophylactically effective amount of the composition of the present disclosure to a subject (an individual) suffering or being likely to suffer the target disease, thereby forming the same amount thereof in the body of the subject. The route of administration of the composition of the present disclosure may encompass all the general routes as long as the composition can arrive at a target tissue, and the composition may be administered orally or parenterally. In addition, the composition of the present disclosure may be administered by using any apparatus that can deliver an active ingredient to a target cell, tissue, or organ.

The term "therapeutically effective amount" of the composition refers to a content of the composition, which is sufficient to provide a therapeutic or prophylactic effect to a subject, to which the composition is to be administered, and thus the term has a meaning encompassing "prophylactically effective amount".

In an embodiment of the present disclosure, the subject is selected from a human, a monkey, a cow, a horse, sheep, a pig, a chicken, a turkey, a quail, a cat, a dog, a mouse, a rat, a rabbit, and a guinea pig, but is not limited thereto. Most specifically, the subject is a human.

The inflammatory bowel disease, which is a target disease of the prevention and method of the present disclosure, is the same as defined with respect to the target disease of the pharmaceutical composition or food composition.

Since the method for preventing or treating inflammatory bowel disease of the present disclosure includes administering the pharmaceutical composition according to an aspect of the present disclosure, the overlapping description with respect to the pharmaceutical composition is omitted to avoid excessive redundancy of the present specification.

### Advantageous Effects of Disclosure

Features and advantages of the present disclosure are summarized as follows.

The mixed extract of Coptis Rhizome and *Schizonepeta tenuifolia* Briquet exhibits effects of preventing or treating inflammatory bowel disease by inhibiting the activity of the inflammatory factor TNF-α, IL-1β, IL-17, IL-23, CCL2, or MIP-2.

Through the above effects, the use of the extract of Coptis Rhizome and *Schizonepeta tenuifolia* Briquet of the present disclosure can provide a pharmaceutical composition for preventing or treating inflammatory bowel disease or a food composition and a feed composition for preventing or alleviating inflammatory bowel disease, each of which can be safely used on the human body without toxicity and side effects.

### Brief Description of Drawings

FIG. 1 shows the disease activity index (DAI) score of day 10 for each administration group in dextran sulfate sodium (DSS)-induced colitis mice.
FIG. 2 shows the histological colitis score for each administration group in DSS-induced colitis mice.
FIG. 3 shows the survival rate (%) for each administration group in dinitrobenzene sulfonic acid (DNBS)-induced colitis mice.
FIG. 4 shows the colon length for each administration group in DNBS-induced colitis mice.
FIG. 5 shows the body weight change rate (%) for 14 days for each administration group in DSS-induced colitis mice.
FIG. 6 shows the DAI score of day 10 for each administration group in DSS--induced colitis mice.
FIG. 7 shows the DAI scores of day 10 for single extract and mixed extract administration groups in DSS-induced colitis mice.
FIG. 8 shows the TNF-α expression levels for single extract and mixed extract administration groups in DSS-induced colitis mice.
FIG. 9 shows the IL-1β expression levels for single extract and mixed extract administration groups in DSS-induced colitis mice.
FIG. 10 shows the IL-17 expression levels for single extract and mixed extract administration groups in DSS-induced colitis mice.
FIG. 11 shows the IL-23 expression levels for single extract and mixed extract administration groups in DSS-induced colitis mice.
FIG. 12 shows the MIP-2 expression levels for single extract and mixed extract administration groups in DSS-induced colitis mice.
FIG. 13 shows the CCL2 expression levels for single extract and mixed extract administration groups in DSS-induced colitis mice.
FIG. 14 shows the NO production amounts for the groups administered single extract and mixed herbal extracts with various mixing ratios in macrophages having inflammation induced by LPS.
FIG. 15 shows the NO production amounts for the groups administered mixed herbal extracts according to the concentration of an extraction solvent in macrophages having inflammation induced by LPS.
FIG. 16 shows the body weight change rate (%) for single extract and mixed herbal extract administration groups in DSS-induced colitis mice.
FIG. 17 shows the DAI scores of day 10 for single extract and mixed herbal extract administration groups in DSS--induced colitis mice.
FIG. 18 shows the colon lengths for single extract and mixed herbal extract administration groups in DSS-induced colitis mice.

### Mode for Carrying Out the Disclosure

Hereinafter, the present disclosure will be described in more detail with reference to exemplary embodiments. These exemplary embodiments are provided only for the purpose of illustrating the present disclosure in more detail, and therefore, according to the purpose of the present disclosure, it would be apparent to a person skilled in the art that these examples are not construed to limit the scope of the present disclosure.

### Examples

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Example 1: Preparation of single herbal extract powders

Twenty-four kinds of herbs in a washed and dried state, Mume Fructus, Terminaliae Fructus, Ecliptae Herba, Dioscoreae Rhizoma, Selaginellae Herba, *Schizonepeta tenuifolia* Briquet, Geranii Herba, Psoraleae Semen, Thujae Orientalis Folium, Sophorae Flos, Sophorae Radix, Coptis Rhizome, Cirsii Herba, Sanguisorbae Radix, Galla Rhois, Arecae Semen, Cimicifugae Rhizoma, Ailanthi Radicis Cortex, Araliae Continentalis Radix, Puerariae Radix, Platycodonis Radix, Schisandrae Fructus, Liriopis Tuber, and Auriculariae Polyporus, were purchased.

Mume Fructus, Terminaliae Fructus, Ecliptae Herba, Dioscoreae Rhizoma, Selaginellae Herba, *Schizonepeta tenuifolia* Briquet, Geranii Herba, Psoraleae Semen, Thujae Orientalis Folium, Sophorae Flos, Sophorae Radix, Coptis Rhizome, Cirsii Herba, Sanguisorbae Radix, Galla Rhois, Arecae Semen, Cimicifugae Rhizoma, Ailanthi Radicis Cortex, Araliae Continentalis Radix, Puerariae Radix, Platycodonis Radix, Schisandrae Fructus, Liriopis Tuber, and Auriculariae Polyporus were separately subjected to extraction with favorable stirring at room temperature for 72 hours after the addition of 70 %(v/v) ethanol solution having a 10-fold weight of each. The extracts were filtered, concentrated under reduced pressure at 50-5°C, and then freeze-dried, to obtain single extract powders for the 24 kinds of herbs, respectively, and yields thereof are shown in Table 1 below.

**TABLE 1**

| Extraction yields of single herbal extracts | |
|---|---|
| **Herb name** | **Extraction yield (%)** |
| Mume Fructus | 21.09 |
| Terminaliae Fructus | 53.90 |
| Ecliptae Herba | 14.51 |
| Dioscoreae Rhizoma | 7.28 |
| Selaginellae Herba | 8.93 |
| *Schizonepeta tenuifolia* Briquet | 9.19 |
| Geranii Herba | 11.53 |
| Psoraleae Semen | 8.35 |
| Thujae Orientalis Folium | 14.81 |
| Sophorae Flos | 26.11 |
| Sophorae Radix | 12.95 |
| Coptis Rhizome | 17.81 |
| Cirsii Herba | 10.58 |
| Sanguisorbae Radix | 33.90 |
| Galla Rhois | 58.27 |
| Arecae Semen | 6.92 |
| Cimicifugae Rhizoma | 19.60 |
| Ailanthi Radicis Cortex | 5.00 |
| Araliae Continentalis Radix | 8.10 |
| Puerariae Radix | 21.79 |
| Platycodonis Radix | 21.56 |
| Schisandrae Fructus | 32.50 |
| Liriopis Tuber | 26.49 |
| Auriculariae Polyporus | 1.90 |

### Example 2: Experiment of effects of single herbal extracts in DSS-induced enteritis model.

After 8-week-old male mice (C57BL/6) were acclimated for one week or longer, the mice were classified into: a normal group; a DSS and distilled water administration group in which Dextran Sulfate Sodium (DSS; 36,000-50,000 MW, MP Biomedicals, USA) was administered to induce ulcerative colitis and then distilled water was administered (negative control); a DSS and 5-aminosalicylic acid (5-ASA; Tokyo Chemical Industry Co., Japan) administration group (positive control group); and groups in which DSS and respective single herbal extracts prepared in Example 1 were administered (experimental groups).

The DSS-induced enteritis model has been known to show similar morphological changes and symptoms to human ulcerative colitis. The mice were fed 100 mL of DSS diluted in drinking water to 2.5%, which was then changed every two days. After the induction by DSS for 5 days, drinking water was changed.

5-ASA, used as a drug for the positive control group, was diluted in drinking water to 25 mg/kg, and administered orally once/day for 14 days from the start date of the experiment. Each single herbal extract was orally administered at a dose of 100 mg/kg once/day for 14 days from the start date of the experiment.

The mice of each group were weighed to calculate weight loss rates, and the feces state and the presence of bloody feces were checked, every day after the start of the experiment. Scoring was conducted for the three indicators according to the standards in Table 2 below.

**TABLE 2**

| Scoring standards for DAI calculation | | | |
|---|---|---|---|
| **Score** | **Body weight loss rate** | **Feces state** | **Bleeding** |
| 0 | < 1 % | Normal | Normal |
| 1 | 1-5 % | - | - |
| 2 | 5-10 % | Watery feces not sticking to the periphery of the anus | Slight bleeding |
| 3 | 10-15 % | - | - |
| 4 | >15 % | Diarrhea sticking to the periphery of the anus | Visible bleeding |

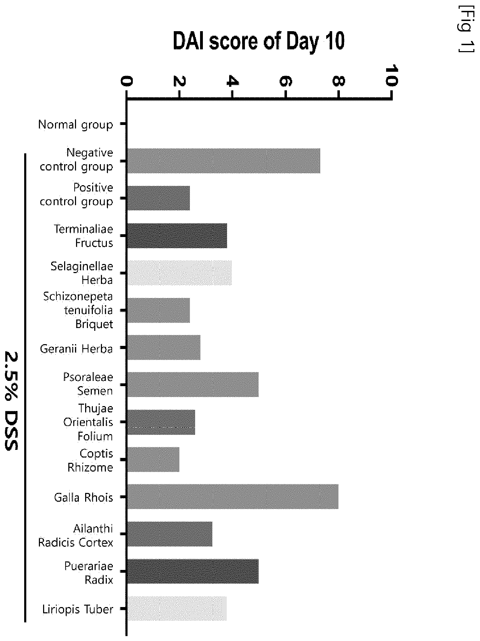

Disease activity index (DAI), which is the sum calculated according to the scoring standards on Table 2 above, is shown in Table 3 below and FIG. 1.

**TABLE 3**

| DAI score for each administration group | |
|---|---|
| **Subject** | **DAI score of Day 10** |
| Normal group | 0.0 |
| Negative control | 7.3 |
| Positive control | 2.4 |
| Terminaliae Fructus | 3.8 |
| Selaginellae Herba | 4.0 |
| *Schizonepeta tenuifolia* Briquet | 2.4 |
| Geranii Herba | 2.8 |
| Psoraleae Semen | 5.0 |
| Thujae Orientalis Folium | 2.6 |
| Coptis Rhizome | 2.0 |
| Galla Rhois | 8.0 |
| Ailanthi Radicis Cortex | 3.3 |
| Puerariae Radix | 5.0 |
| Liriopis Tuber | 3.8 |

As shown in Table 3 above, the DAI score of the group administered Coptis Rhizome was 2.0, the lowest value, and the DAI score of the group administered *Schizonepeta tenuifolia* Briquet was 2.4, the same as that of the positive control group.

On the last day of the experiment, the mice were sacrificed with carbon dioxide gas, and the colon site was excised. The colon length was measured from the excised colon, and then the state of the intestinal mucosa was investigated by incision in the longitudinal direction.

The measured colon distribution is shown in Table 4 below.

**TABLE 4**

| **Subject** | **Colon length (cm)** |
|---|---|
| Normal group | 7.2 |
| Negative control group | 3.9 |
| Positive control | 5.6 |
| Terminaliae Fructus | 5.5 |
| Selaginellae Herba | 5.3 |
| *Schizonepeta tenuifolia* Briquet | 5.8 |
| Geranii Herba | 6.0 |
| Psoraleae Semen | 5.4 |
| Thujae Orientalis Folium | 5.5 |
| Coptis Rhizome | 6.1 |
| Galla Rhois | 4.2 |
| Ailanthi Radicis Cortex | 5.9 |
| Puerariae Radix | 6.1 |
| Liriopis Tuber | 4.9 |

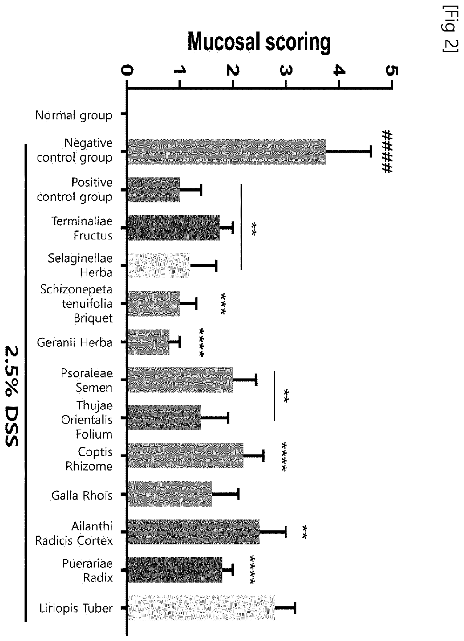

The histological colitis score was calculated by measuring the lengths of the mucosal ulcer site and the damaged mucosal layer in the colon tissue and averaging the values. The results are shown in Table 5 below and FIG. 2.

**TABLE 5**

| Histological colitis score of each administration group | |
|---|---|
| **Subject** | **Histological colitis score** |
| Normal group | 0.00 |
| Negative control group | 3.75 |
| Positive control | 1.00 |
| Terminaliae Fructus | 1.75 |
| Selaginellae Herba | 1.20 |
| *Schizonepeta tenuifolia* Briquet | 1.00 |
| Geranii Herba | 0.80 |
| Psoraleae Semen | 2.00 |
| Thujae Orientalis Folium | 1.40 |
| Coptis Rhizome | 2.20 |
| Galla Rhois | 1.60 |
| Ailanthi Radicis Cortex | 2.50 |
| Puerariae Radix | 1.80 |
| Liriopis Tuber | 2.80 |

As shown in Table 5 above, the histological colitis score of the group administered Geranii Herba was lowest, 0.80, and then the histological colitis score of the group administered *Schizonepeta tenuifolia* Briquet was 1.00, which was the same as that of the positive control group.

Ultimately, as a result of inducing enteritis by DSS administration, the groups administered the extracts of 11 kinds of herbs, Terminaliae Fructus, Selaginellae Herba, *Schizonepeta tenuifolia* Briquet, Geranii Herba, Psoraleae Semen, Thujae Orientalis Folium, Coptis Rhizome, Galla Rhois, Ailanthi Radicis Cortex, Puerariae Radix, and Liriopis Tuber showed an improvement effect in DAI score or histological colitis score compared with the negative control group.

### Example 3: Experiment of effects of single herbal extracts on DNBS-induced enteritis model

After 8-week-old male mice (C57BL/6) were acclimated for one week or longer, the mice were classified into: a normal group; a DNBS and distilled water administration group in which dinitrobenzene sulfonic acid (DNBS; Sigma-Aldrich, USA) was administered to induce inflammatory bowel disease and then distilled water was administered (negative control); a DNBS and 5-ASA administration group (positive control group); and groups in which DNBS and extracts of 11 kinds of herbs (Terminaliae Fructus, Selaginellae Herba, *Schizonepeta tenuifolia* Briquet, Geranii Herba, Psoraleae Semen, Thujae Orientalis Folium, Coptis Rhizome, Galla Rhois, Ailanthi Radicis Cortex, Puerariae Radix, and Liriopis Tuber) selected in Example 2 (experimental groups).

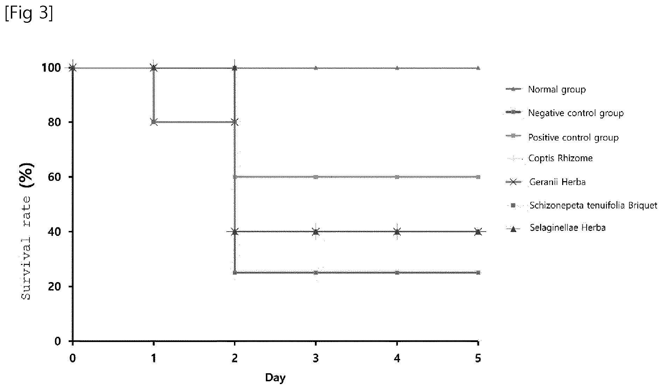

The DNBS-induced enteritis model is known to be closely related with human Crohn's disease in that T cell-mediated immune response is induced. DNBS in 50% ethanol/distilled water at 40 mg/mL was prepared, and 0.1 mL was slowly injected into the rectum of the mouse by using a catheter. 5-ASA, used as a drug for the positive control group, was diluted in drinking water to 25 mg/kg, and administered orally once/day for 5 days from the start date of the experiment. Each single herbal extract was orally administered at a dose of 100 mg/kg once/day for 5 days from the start date of the experiment, and the survival rate was checked every day. The results are shown in Table 6 below and FIG. 3.

**TABLE 6**

| Survival rate (%) of each administration group | |
|---|---|
| **Subject** | **Survival rate (%)** |
| Normal group | 100 |
| Negative control group | 25 |
| Positive control | 60 |
| Coptis Rhizome | 40 |
| Geranii Herba | 40 |
| Selaginellae Herba | 40 |
| *Schizonepeta tenuifolia* Briquet | 40 |

As shown in Table 6 above, most of the administration groups showed individual death due to DNBS administration, but all the groups administered the extracts of four kinds of herbs showed a survival rate of 40%.

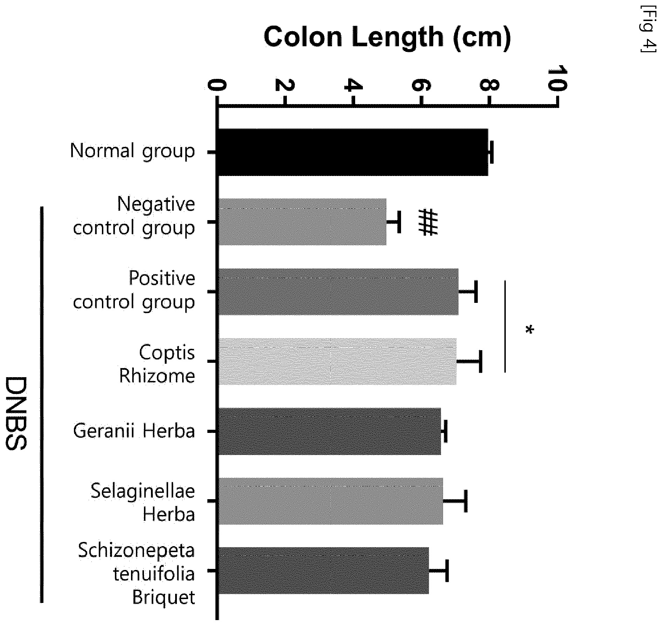

On the last day of the experiment, the mice were sacrificed with carbon dioxide gas, and the colon site was excised, and the colon length was measured. The results are shown in Table 7 below and FIG. 4.

**TABLE 7**

| Colon length for each administration group | |
|---|---|
| **Subject** | **Colon length (cm)** |
| Normal group | 7.96 |
| Negative control group | 4.98 |
| Positive control | 7.10 |
| Coptis Rhizome | 7.04 |
| Geranii Herba | 6.58 |
| Selaginellae Herba | 6.64 |
| *Schizonepeta tenuifolia* Briquet | 6.23 |

As shown in Table 7 above, as a result of measuring the colon length, the groups administered Coptis Rhizome, Geranii Herba, *Schizonepeta tenuifolia* Briquet, and Selaginellae Herba showed a reduction in the degree of colon length decrease compared with the negative control group.

It can be ultimately seen that Coptis Rhizome, Geranii Herba, *Schizonepeta tenuifolia* Briquet, and Selaginellae Herba had an excellent effect in the alleviation or treatment of enteritis.

### Example 4: Experiment of effects of single herbal extracts with different concentration

After 8-week-old male mice (C57BL/6) were acclimated for one week or longer, the mice were classified into: a normal group; a DSS and distilled water administration group (negative control); a DSS and 5-ASA administration group (positive control group); and groups in which DSS and extracts of four kinds of herbs (Coptis Rhizome, Geranii Herba, *Schizonepeta tenuifolia* Briquet, and Selaginellae Herba) selected in Example 1 (experimental groups).

The mice were fed 100 mL of DSS diluted in drinking water to 2.5%, which was then changed every two days. To evaluate the effects of single herbal extracts with different concentrations, DSS was administered for a total of 6 days, and then drinking water was changed.

5-ASA was distilled in drinking water to 25 mg/kg and orally administered.

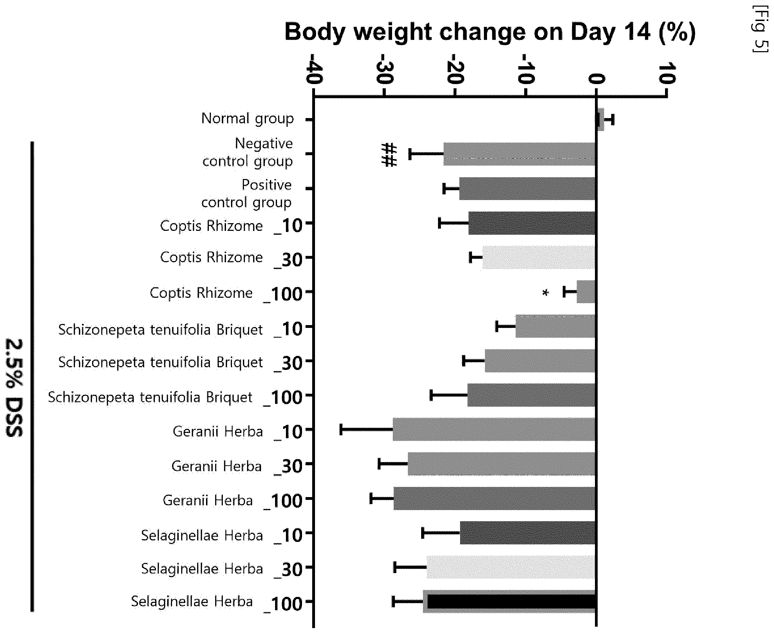

Each single herbal extract was orally administered at doses of 10, 30, and 100 mg/kg once/day for 14 days from the start date of the experiment, and the body weight change, feces state, and the presence of bloody feces were checked every day. The body weight change is shown in Table 8 and FIG. 5.

**TABLE 8**

| Body weight change rate for each administration group with different concentrations | |
|---|---|
| **Subject** | **Body weight change rate for 14 days (%)** |
| Normal group | 1.16 |
| Negative control group | -21.65 |
| Positive control | -19.38 |
| Coptis Rhizome 10 | -18.08 |
| Coptis Rhizome 30 | -16.12 |
| Coptis Rhizome 100 | -2.76 |
| *Schizonepeta tenuifolia* Briquet 10 | -11.43 |
| *Schizonepeta tenuifolia* Briquet 30 | -15.80 |
| *Schizonepeta tenuifolia* Briquet 100 | -18.20 |
| Geranii Herba 10 | -28.80 |
| Geranii Herba 30 | -26.70 |
| Geranii Herba 100 | -28.68 |
| Selaginellae Herba 10 | -19.34 |
| Selaginellae Herba 30 | -24.02 |
| Selaginellae Herba 100 | -24.56 |

As shown in Table 8 above, the body weight change rate decreased with the increase in concentration in the groups administered Coptis Rhizome. However, the body weight change rate rather increased with the increase in concentration in the groups administered *Schizonepeta tenuifolia* Briquet, and there was no significant difference in body weight change rate according to the concentration in the groups administered Geranii Herba and Selaginellae Herba.

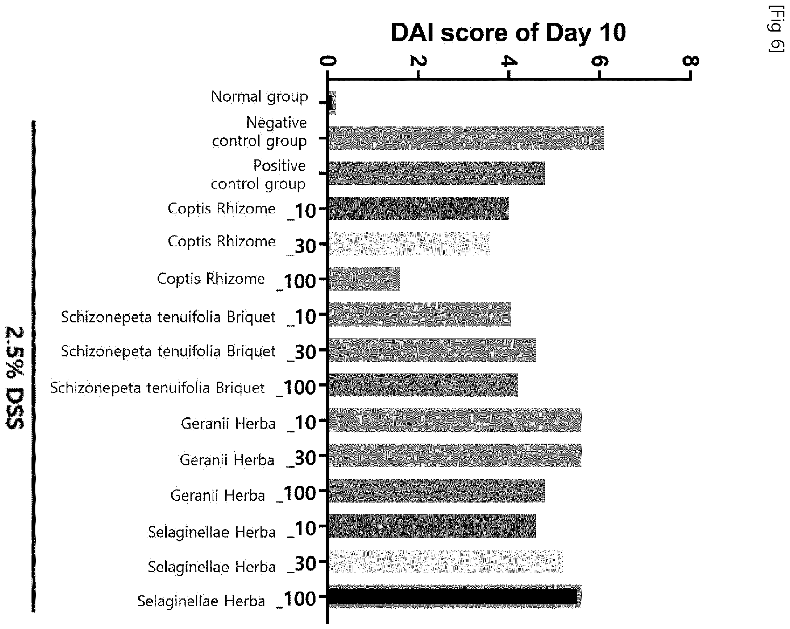

In addition, DAI scores were calculated based on the results of the body weight change, feces state, and the presence of bloody feces, and the results are shown in Table 9 below and FIG. 6.

**TABLE 9**

| DAI score for each administration group | |
|---|---|
| **Subject** | **DAI score of Day 10** |
| Normal group | 0.2 |
| Negative control group | 6.1 |
| Positive control | 4.8 |
| Coptis Rhizome 10 | 4.0 |
| Coptis Rhizome 30 | 3.6 |
| Coptis Rhizome 100 | 1.6 |
| *Schizonepeta tenuifolia* Briquet 10 | 4.1 |
| *Schizonepeta tenuifolia* Briquet 30 | 4.6 |
| *Schizonepeta tenuifolia* Briquet 100 | 4.2 |
| Geranii Herba 10 | 5.6 |
| Geranii Herba 30 | 5.6 |
| Geranii Herba 100 | 4.8 |
| Selaginellae Herba 10 | 4.6 |
| Selaginellae Herba 30 | 5.2 |
| Selaginellae Herba 100 | 5.6 |

As shown in Table 9 above, the DAI score significantly decreased with the increase in concentration in the groups administered Coptis Rhizome. However, there was no significant difference in DAI score according to the concentration in the groups administered *Schizonepeta tenuifolia* Briquet, Geranii Herba and Selaginellae Herba.

### Example 5: Comparison of effects between single extracts and mixed extracts

After 8-week-old male mice (C57BL/6) were acclimated for one week or longer, the mice were classified into: a normal group; a DSS administration group, a DSS and distilled water administration group (negative control); a DSS and 5-ASA administration group (positive control group); and a group administered DSS and Coptis Rhizome 100 mg/kg, a group administered DSS and *Schizonepeta tenuifolia* Briquet 10 mg/kg, and a group administered DSS, Coptis Rhizome 100 mg/kg, and *Schizonepeta tenuifolia* Briquet 10 mg/kg (mixed extract administration group).

The mice were fed 100 mL of DSS diluted in drinking water to 2.5%, which was then changed every two days. After DSS administration for 5 days, drinking water was changed.

5-ASA, used as a drug for the positive control group, was diluted in drinking water to 25 mg/kg, and orally administered.

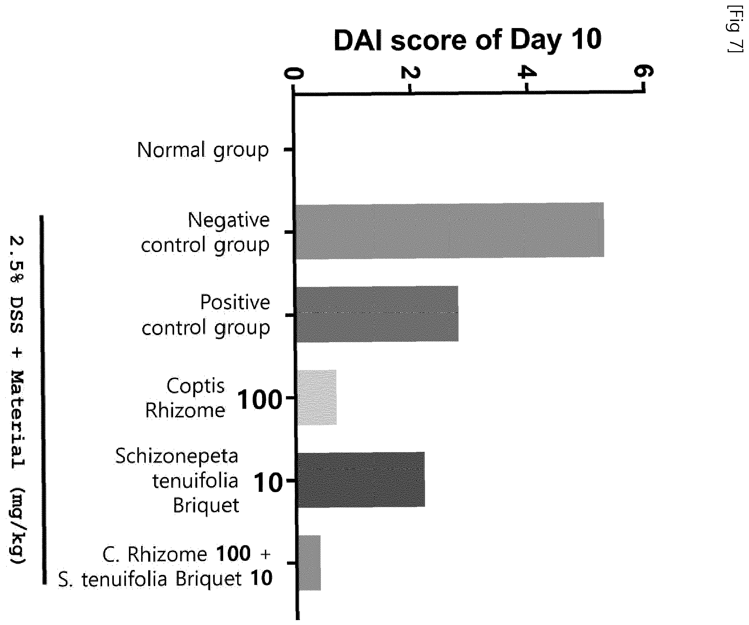

Coptis Rhizome 100 mg/kg, *Schizonepeta tenuifolia* Briquet 10 mg/kg, and a 10:1 mixture of Coptis Rhizome and *Schizonepeta tenuifolia* Briquet at a dose of 110 mg/kg were orally administered once/day for 14 days, and the body weight change rate, feces state, and the presence of bloody feces were checked every day. DAI scores were calculated based on the experimental results, and shown in Table 10 below and FIG. 7.

**TABLE 10**

| DAI score of Day 10 for each administration group | |
|---|---|
| **Subject** | **DAI score of Day 10** |
| Normal group | 0.0 |
| Negative control group | 5.3 |
| Positive control | 2.8 |
| Coptis Rhizome 100 | 0.7 |
| *Schizonepeta tenuifolia* Briquet 10 | 2.2 |
| Coptis Rhizome 100 and *Schizonepeta tenuifolia* Briquet 10 | 0.4 |

As shown in Table 10 above, the single Coptis Rhizome 100 mg/kg single administration group, the *Schizonepeta tenuifolia* Briquet 10 mg/kg single administration group, and the Coptis Rhizome and *Schizonepeta tenuifolia* Briquet mixed administration group all showed lower DAI scores than the positive control group. It was also identified that the mixed administration group had a lower DAI score compared with single administration groups.

### Example 6: Measurement of expression changes of inflammatory factors TNF-α, IL-1β, IL-17, IL-23, CCL2, and MIP-2

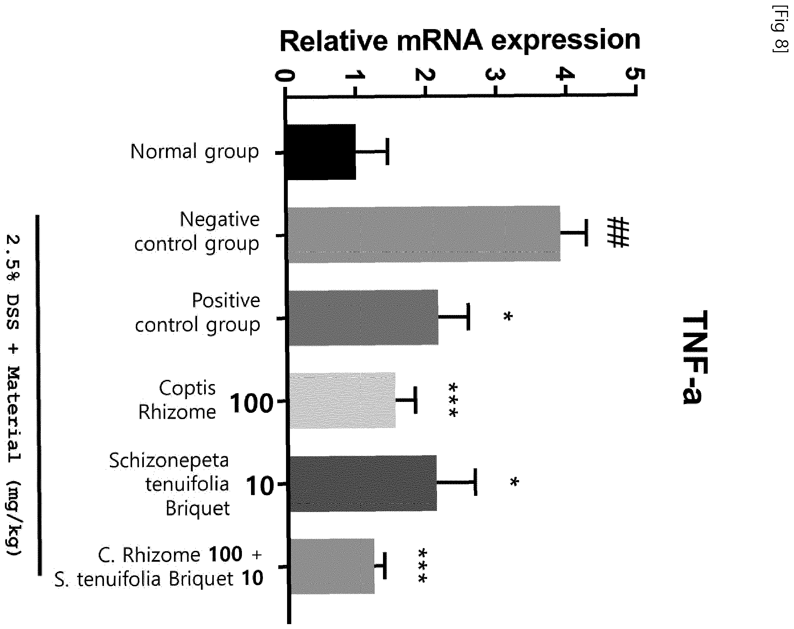

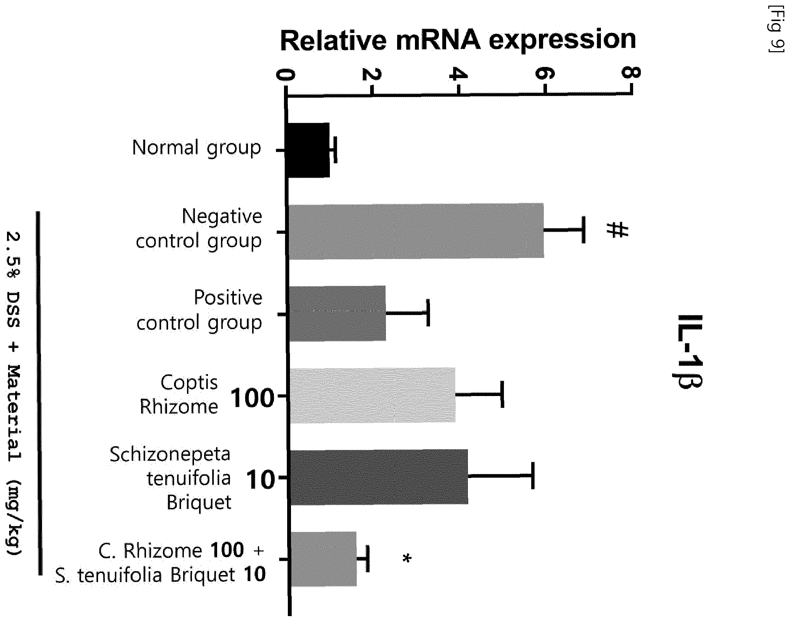

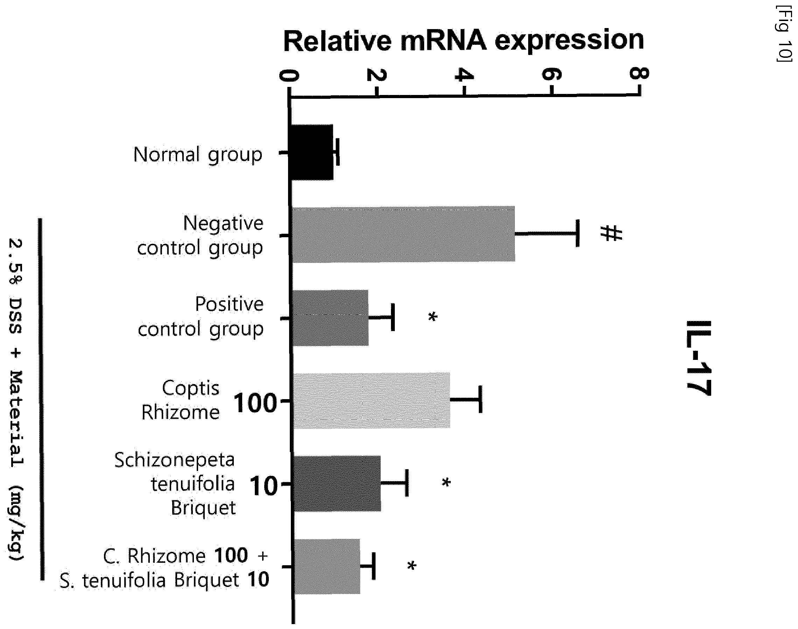

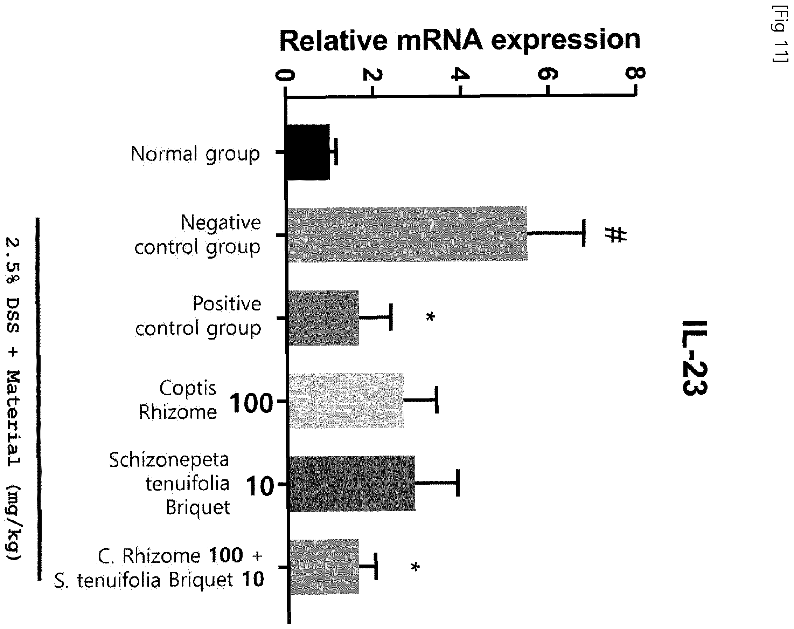

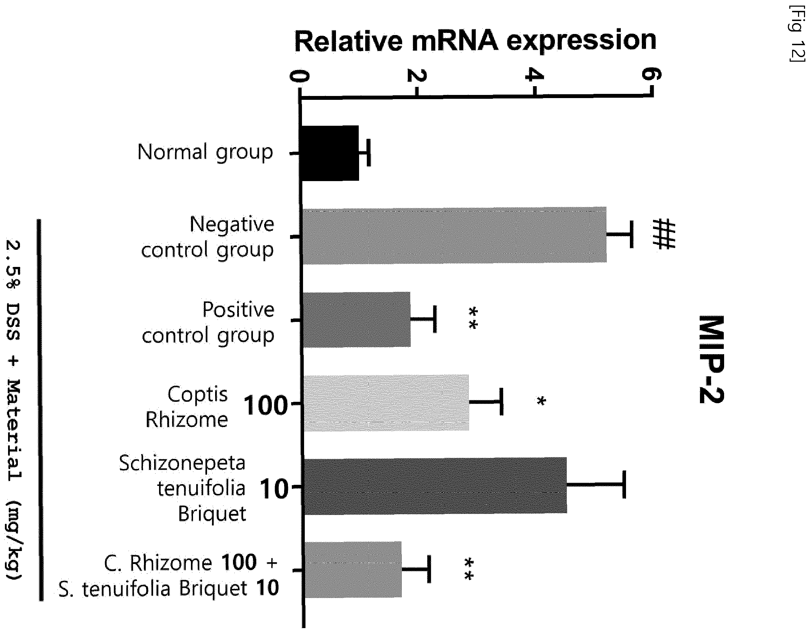

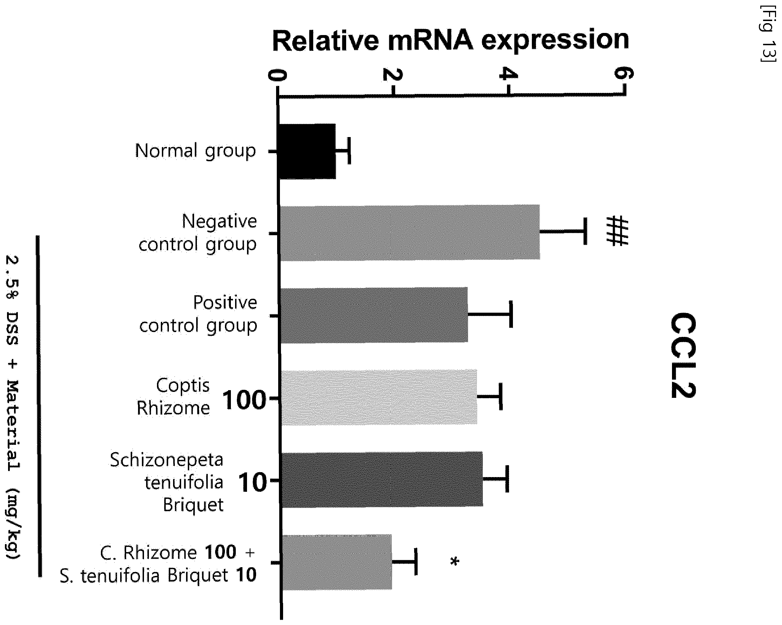

RNA was isolated from the colon of the mice collected in Example 5 by using TRIzol (Invitrogen, USA). Thereafter, quantitative PCR through primers specific to the inflammatory factors TNF-α, IL-1β, IL-17, IL-23, CCL2, and MIP-2, and SYBR green (Takara, Japan) was performed using cDNA obtained by performing RT-PCR. The expression levels of RNA obtained therefrom were expressed as a relative change compared with the untreated group (normal group) by using GAPDH mRNA as the standard gene. The experimental results are shown in Tables 11 to 16 below and FIGS. 8 to 13. The primer sequences for mouse genes used in the experiments are shown in Table 17 below.

**TABLE 11**

| | |
|---|---|
| TNF-α expression level (relative value) for each administration group | |

| **Subject** | **TNF-α expression level** |
|---|---|
| Normal group | 1.00 |
| Negative control group | 3.91 |
| Positive control | 2.16 |
| Coptis Rhizome 100 | 1.54 |
| *Schizonepeta tenuifolia* Briquet 10 | 2.12 |
| Coptis Rhizome 100 and *Schizonepeta tenuifolia* Briquet 10 | 1.22 |

**TABLE 12**

| IL-1β expression level (relative value) for each administration group | |
|---|---|
| **Subject** | **IL-1β expression level** |
| Normal group | 1.00 |
| Negative control group | 5.94 |
| Positive control | 2.28 |
| Coptis Rhizome 100 | 3.87 |
| *Schizonepeta tenuifolia* Briquet 10 | 4.15 |
| Coptis Rhizome 100 and *Schizonepeta tenuifolia* Briquet 10 | 1.57 |

**TABLE 13**

| | |
|---|---|
| IL-17 expression level (relative value) for each administration group | |

| **Subject** | **IL-17 expression level** |
|---|---|
| Normal group | 1.00 |
| Negative control group | 5.12 |
| Positive control | 1.77 |
| Coptis Rhizome 100 | 3.62 |
| *Schizonepeta tenuifolia* Briquet 10 | 2.03 |
| Coptis Rhizome 100 and *Schizonepeta tenuifolia* Briquet 10 | 1.55 |

**TABLE 14**

| | |
|---|---|
| IL-23 expression level (relative value) for each administration group | |

| **Subject** | **IL-23 expression level** |
|---|---|
| Normal group | 1.00 |
| Negative control group | 5.51 |
| Positive control | 1.65 |
| Coptis Rhizome 100 | 2.65 |
| *Schizonepeta tenuifolia* Briquet 10 | 2.91 |
| Coptis Rhizome 100 and *Schizonepeta tenuifolia* Briquet 10 | 1.60 |

**TABLE 15**

| | |
|---|---|
| MIP-2 expression level (relative value) for each administration group | |

| **Subject** | **MIP-2 expression level** |
|---|---|
| Normal group | 1.00 |
| Negative control group | 5.21 |
| Positive control | 1.86 |
| Coptis Rhizome 100 | 2.85 |
| *Schizonepeta tenuifolia* Briquet 10 | 4.50 |
| Coptis Rhizome 100 and *Schizonepeta tenuifolia* Briquet 10 | 1. 68 |

**TABLE 16**

| | |
|---|---|
| CCL2 expression level (relative value) for each administration group | |

| **Subject** | **CCL2 expression level** |
|---|---|
| Normal group | 1.00 |
| Negative control group | 4.51 |
| Positive control | 3.26 |
| Coptis Rhizome 100 | 3.42 |
| *Schizonepeta tenuifolia* Briquet 10 | 3.51 |
| Coptis Rhizome 100 and *Schizonepeta tenuifolia* Briquet 10 | 1.92 |

**TABLE 17**

| | | |
|---|---|---|
| Primer sequences for mouse genes | | |

| **Gene** | **Direction** | **Nucleotide sequence (5' to 3)** |
|---|---|---|
| GAPDH | Forward | AGCCTCGTCCCGTAGACAA (SEQ ID NO: 1) |
| | Reverse | AATCTCCACTTTGCCACTGC (SEQ ID NO: 2) |
| TNF-α | Forward | GCCTCTTCTCATTCCTGCTTG (SEQ ID NO: 3) |
| | Reverse | CTGATGAGAGGGAGGCCATT (SEQ ID NO: 4) |
| IL-1β | Forward | TGTGCAAGTGTCTGAAGCAGC (SEQ ID NO: 5) |
| | Reverse | TGGAAGCAGCCCTTCATCTT (SEQ ID NO: 6) |
| IL-23 | Forward | GCTGTGCCTAGGAGTAGCAG (SEQ ID NO: 7) |
| | Reverse | TGGCTGTTGTCCTTGAGTCC (SEQ ID NO: 8) |
| CCL2 | Forward | TCCCACTCACCTGCTGCTACTCA (SEQ ID NO: 9) |
| | Reverse | GCTTCTTTGGGACACCTGCTG (SEQ ID NO: 10) |
| MIP-2 | Forward | CCCTGCCAAGGGTTGACTTC (SEQ ID NO: 11) |
| | Reverse | GCAAACTTTTTGACCGCCCT (SEQ ID NO: 12) |
| IL-17 | Forward | TCTCCACCGCAATGAAGACC (SEQ ID NO: 13) |
| | Reverse | CACACCCACCAGCATCTTCT (SEQ ID NO: 14) |

As shown in Tables 11 to 16, the mixed administration group showed lower values in expression levels of the inflammatory factors TNF-α, IL-1β, IL-17, IL-23, CCL2, and MIP-2 compared with the single administration groups. Ultimately, it can be seen that the mixed administration group is excellent in inflammation inhibitory effect compared with single administration groups.

### Example 7: Preparation of mixed herbal extract powders

To prepare mixed herbal extract powders, washed and dried Coptis Rhizome and *Schizonepeta tenuifolia* Briquet (Schizonepetae Spica and Schizonepetae Herba) were used. Coptis Rhizome and *Schizonepeta tenuifolia* Briquet were mixed at weight ratios (w/w) shown in Table 18, and a 70 %(v/v) ethanol aqueous solution having a 10-fold volume was added thereto, followed by stirring at room temperature for 72 hours, and then extracts were obtained. Thereafter, the respective extracts were filtered, concentrated at 50 to 65°C, and freeze-dried, thereby obtaining powder-type mixed herbal extracts, and yields thereof are shown as follows.

**TABLE 18**

| Yields of extracts with different mixing ratios | | | |
|---|---|---|---|
| **Classification** | **Coptis Rhizome** | ***Schizonepeta tenuifolia* Briquet** | **Extraction yield (%)** |
| Example 7-1 | 1 | 1 | 12.25 |
| Example 7-2 | 2 | 1 | 14.05 |
| Example 7-3 | 4 | 1 | 15.97 |
| Example 7-4 | 8 | 1 | 16.78 |
| Example 7-5 | 10 | 1 | 16.75 |

### Example 8: Preparation of mixed herbal extract powders according to different extraction solvents

Washed and dried Coptis Rhizome and *Schizonepeta tenuifolia* Briquet (Schizonepetae Spica and Schizonepetae Herba) were used. Coptis Rhizome and *Schizonepeta tenuifolia* Briquet were mixed at a weight ratio (w/w) of 10:1, and then 25, 50, 70, and 95 %(v/v) ethanol aqueous solutions having a 10-fold volume were added thereto, followed by stirring at room temperature for 72 hours, and then extracts were obtained. Coptis Rhizome and *Schizonepeta tenuifolia* Briquet were mixed at a weight ratio (w/w) of 10:1, and then distilled water having a 10-fold volume was added thereto, followed by extraction under reflux at a temperature at which 90-95°C was maintained, thereby obtaining an extract. Thereafter, the respective extracts were filtered, concentrated at 50 to 65°C, and freeze-dried, thereby obtaining powder-type mixed herbal extracts, and yields thereof are shown as follows.

**TABLE 19**

| Extraction yields of mixed herbal extracts according to different extraction solvents | | | |
|---|---|---|---|
| **Extraction solvent** | **Coptis Rhizome** | ***Schizonepeta tenuifolia* Briquet** | **Extraction yield (%)** |
| water extraction | 10 | 1 | 16.06 |
| EtOH 25% | 10 | 1 | 18.68 |
| EtOH 50% | 10 | 1 | 21.29 |
| EtOH 70% | 10 | 1 | 20.56 |
| EtOH 95% | 10 | 1 | 4.37 |

### Example 9: Experiment of effects of single and mixed herbal extracts on LPS-induced nitric oxide (NO) production of macrophage cell line

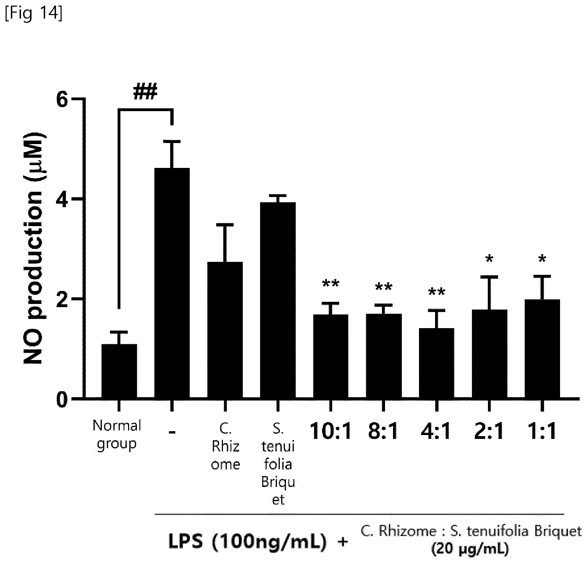

The mouse macrophage cell line Raw264.7 cells (ATCC, USA) were cultured at 37°C in a 5% CO₂ incubator using RPMI media (Invitrogen, USA) containing 10% FBS. The cells were prepared on a 24-well plate at 5×10⁴ cells per well, and stabilized. After 24 hours, the cell supernatant was removed, and then the cells were treated with the single herbal extracts in Example 1 and the mixed herbal extracts in Example 7 at a concentration of 20 µg/mL. After 30 minutes, the cells were further treated with 100 ng/mL LPS. After 24 hours, the cell supernatant was collected, and the Griess test for measuring the change in NO production was performed. The concentrations of NO were calculated using the standard curve according to the concentration of sodium nitrite (NaNO₂). The results are shown in Table 20 below and FIG. 14.

As shown in FIG. 14, the production of the inflammatory factor NO increased to a level of 4.62 µM by LPS treatment in macrophages (negative control group). The NO concentrations decreased to 2.75 and 3.93 µM in the experimental groups treated with the single herbal extracts together with LPS, respectively. However, the NO concentrations decreased to 1.69, 1.71, 1.41, 1.79, and 1.99 µM in the experimental groups treated with the mixed herbal extracts together with LPS, respectively, indicating that the treatment with mixed herbal extracts shows a significantly high inhibitory ability on NO production compared with the treatment with single herbal extracts. It can be therefore identified that the mixed herbal extracts of the present disclosure showed an excellent anti-inflammatory effect.

**TABLE 20**

| NO production of each administration group | |
|---|---|
| **Subject** | **NO production (µM)** |
| Normal group | 1.10 |
| Negative control group | 4.62 |
| Coptis Rhizome | 2.75 |
| *Schizonepeta tenuifolia* Briquet | 3.93 |
| Coptis *Rhizome:Schizonepeta tenuifolia* Briquet = 10:1 | 1.69 |
| Coptis *Rhizome:Schizonepeta tenuifolia* Briquet = 8:1 | 1.71 |
| Coptis *Rhizome:Schizonepeta tenuifolia* Briquet = 4:1 | 1.41 |
| Coptis *Rhizome:Schizonepeta* | 1.79 |
| *tenuifolia* Briquet = 2:1 | |
| Coptis *Rhizome:Schizonepeta tenuifolia* Briquet = 1:1 | 1.99 |

### Example 10: Experiment of effects of mixed herbal extracts according to hot water and extraction solvent with different concentrations on LPS-induced nitric oxide (NO) production of macrophage cell line

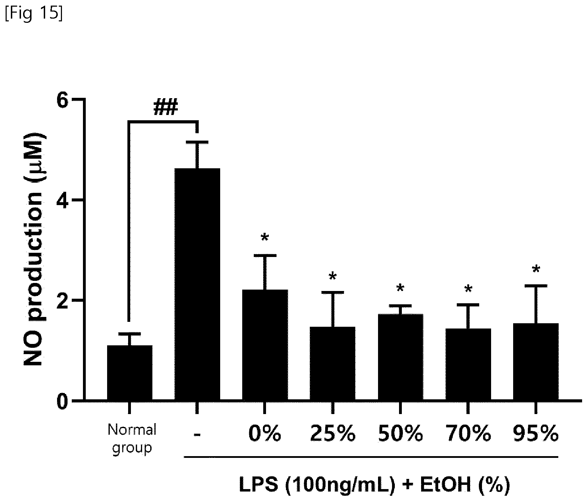

The mouse macrophage cell line Raw264.7 cells (ATCC, USA) were cultured at 37°C in a 5% CO₂ incubator using RPMI media (Invitrogen, USA) containing 10% FBS. The cells were prepared on a 24-well plate at 5×10⁴ cells per well, and stabilized. After 24 hours, the cell supernatant was removed, and then the cells were treated with mixed herbal extracts according to hot water and extraction solvent (ethanol) with different concentrations in Example 8 at a concentration of 20 µg/mL. After 30 minutes, the cells were further treated with 100 ng/mL LPS. After 24 hours, the cell supernatant was collected, and the Griess test for measuring the change in NO production was performed. The concentrations of NO were calculated using the standard curve according to the concentration of sodium nitrite (NaNO₂). The results are shown in Table 21 below and FIG. 15.

As shown in FIG. 15, the production of the inflammatory factor NO increased to a level of 4.62 µM by LPS treatment in macrophages (negative control group). The NO concentrations were measured to be 2.22, 1.48, 1.73, 1.45, and 1.55 µM in the experimental groups treated with hot water and an extraction solvent (ethanol) with different concentrations, respectively. A significant inhibitory ability on NO production was identified in all the extraction experimental groups.

**TABLE 21**

| NO production of each administration group | |
|---|---|
| **Subject** | **NO production (µM)** |
| Normal group | 1.10 |
| Negative control group | 4.62 |
| Ethanol 0% (water extraction) | 2.22 |
| Ethanol 25% | 1.48 |
| Ethanol 50% | 1.73 |
| Ethanol 70% | 1.45 |
| Ethanol 95% | 1.55 |

### Example 11: Experiment of effects of single and mixed herbal extracts on DSS-induced enteritis model

After 8-week-old male mice (C57BL/6) were acclimated for one week or longer, the mice were classified into: a normal group;
a DSS and distilled water administration group in which Dextran Sulfate Sodium (DSS; 36,000-50,000 MW, MP Biomedicals, USA) was administered to induce ulcerative colitis and then distilled water was administered (negative control); a DSS and 5-aminosalicylic acid (5-ASA; Tokyo Chemical Industry Co., Japan) administration group (positive control group); and groups in which together with DSS, Coptis Rhizome and *Schizonepeta tenuifolia* Briquet extracts prepared in Example 1 and mixed herbal extracts prepared in Example 7 were administered (experimental groups).

The DSS-induced enteritis model has been known to show similar morphological changes and symptoms to human ulcerative colitis. The mice were fed 100 mL of DSS diluted in drinking water to 2.5%, which was then changed every two days. After the induction by DSS for 5 days, drinking water was changed.

5-ASA, used as a drug for the positive control group, was diluted in drinking water to 25 mg/kg, and administered orally once/day for 10 days from the start date of the experiment. Each mixed herbal extract was orally administered at a dose of 50 mg/kg once/day for 10 days from the start date of the experiment.

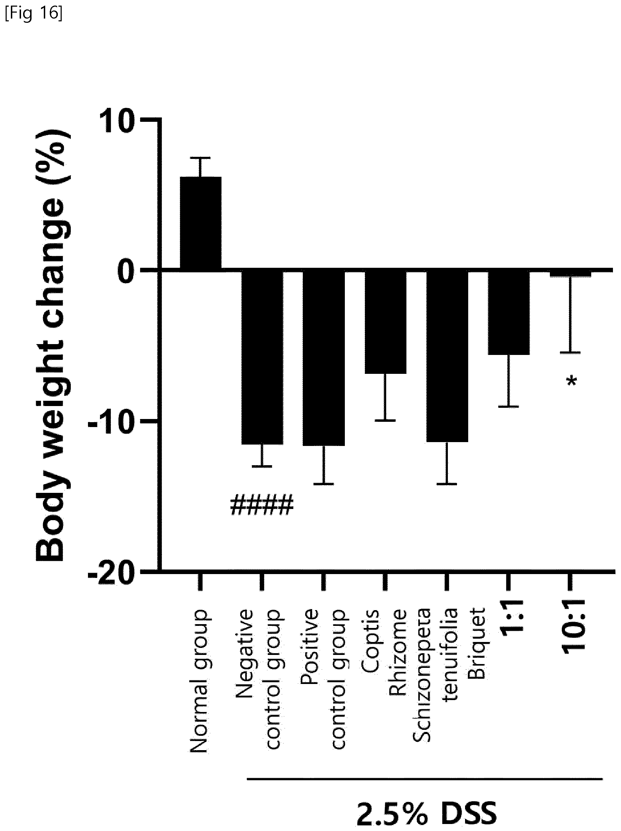

The mice of each group were weighed to calculate weight loss rates every day after the start of the experiment. The body weight changes are shown in Table 22 below and FIG. 16.

**TABLE 22**

| Body weight change rate of each administration group | |
|---|---|
| **Subject** | **Body weight change rate for 10 days (%)** |
| Normal group | 6.24 |
| Negative control group | -11.55 |
| Positive control | -11.66 |
| Coptis Rhizome | -6.83 |
| *Schizonepeta tenuifolia* Briquet | -11.4 |
| Coptis *Rhizome:Schizonepeta tenuifolia* Briquet = 1:1 | -5.63 |
| Coptis *Rhizome:Schizonepeta tenuifolia* Briquet = 10:1 | -0.44 |

As shown in Table 22 above, the body weight change rates in the groups administered mixed herbal extracts of Coptis *Rhizome:Schizonepeta tenuifolia* Briquet at ratios of 1:1 and 10:1 had lower values than those in the Coptis Rhizome single extract administration group, the *Schizonepeta tenuifolia* Briquet single extract administration group, and the positive control group. Especially, a significantly lower value in body weight change rate was observed in the administration of the mixed herbal extract of Coptis Rhizome and *Schizonepeta tenuifolia* Briquet at a ratio of 10:1 than the administration of the mixed herbal extract thereof at a ratio of 1:1.

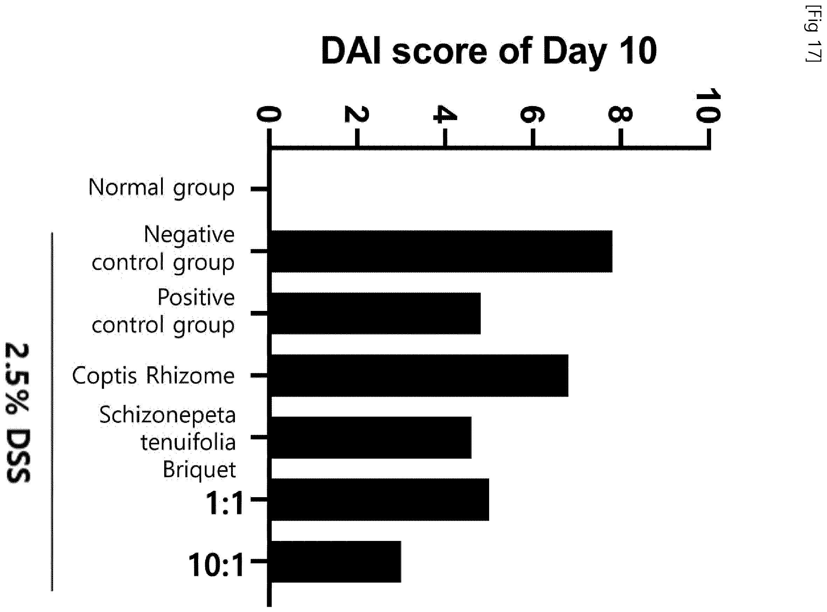

In addition, DAI scores were calculated based on the results of the body weight change, feces state, and the presence of bloody feces, and the results are shown in Table 23 below and FIG. 17.

**TABLE 23**

| DAI score for each administration group | |
|---|---|
| **Subject** | **DAI score of DAY 10** |
| Normal group | 0.0 |
| Negative control group | 7.8 |
| Positive control | 4.8 |
| Coptis Rhizome | 6.8 |
| *Schizonepeta tenuifolia* Briquet | 4.6 |
| Coptis *Rhizome:Schizonepeta tenuifolia* Briquet = 1:1 | 5.0 |
| Coptis *Rhizome:Schizonepeta tenuifolia* Briquet = 10:1 | 3.0 |

As shown in Table 23 above, the DAI score in the group administered the mixed herbal extract of Coptis Rhizome and *Schizonepeta tenuifolia* Briquet at a ratio of 1:1 was lower than that in the Coptis Rhizome single extract group, and similar to those in the positive control group and the *Schizonepeta tenuifolia* Briquet single extract administration group. The DAI score in the group administered the mixed herbal extract of Coptis *Rhizome:Schizonepeta tenuifolia* Briquet at a ratio of and 10:1 had a lower value than those in the Coptis Rhizome single extract administration group, the *Schizonepeta tenuifolia* Briquet single extract administration group, and the positive control group.

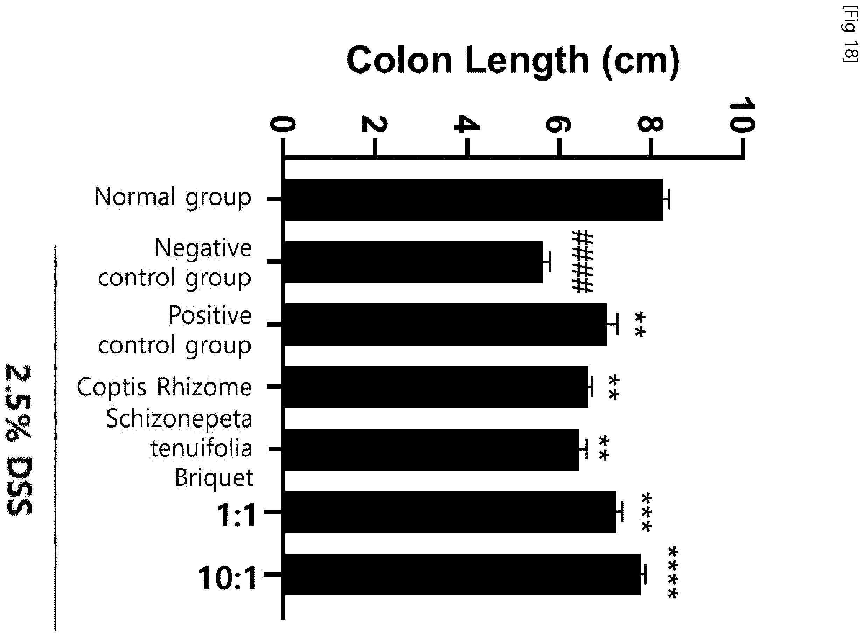

On the last day of the experiment, the mice were sacrificed with carbon dioxide gas, and the colon site was excised. The colon length was measured from the excised colon, and the results are shown in Table 24 below and FIG. 18.

**TABLE 24**

| Colon length of each administration group | |
|---|---|
| **Subject** | **Colon length (cm)** |
| Normal group | 8.26 |
| Negative control group | 5.65 |
| Positive control | 7.04 |
| Coptis Rhizome | 6.63 |
| *Schizonepeta tenuifolia* Briquet | 6.44 |
| Coptis *Rhizome:Schizonepeta tenuifolia* Briquet = 1:1 | 7.25 |
| Coptis *Rhizome:Schizonepeta tenuifolia* Briquet = 10:1 | 7.78 |

As shown in Table 24 above, the colon lengths in the groups administered mixed herbal extracts of Coptis *Rhizome:Schizonepeta tenuifolia* Briquet at ratios of 1:1 and 10:1 had higher values than those in the Coptis Rhizome single extract administration group, the *Schizonepeta tenuifolia* Briquet single extract administration group, and the positive control group. Especially, a significantly high value in colon length was shown in the administration of the mixed herbal extract of Coptis Rhizome and *Schizonepeta tenuifolia* Briquet at a ratio of 10:1 than the administration of the mixed herbal extract thereof at a ratio of 1:1.

Ultimately, as a result of inducing enteritis by DSS administration, the groups administered the mixed herbal extracts of Coptis Rhizome and *Schizonepeta tenuifolia* Briquet showed an improvement effect in body weight change rate, DAI score, or colon length compared with the single herbal extract administration groups.

Hereinafter, formulation examples of the composition containing the extract of the present disclosure will be described, but the present disclosure is not limited thereto and these examples are to specifically explain the present disclosure.

### Formulation examples

### Formulation Example 1. Preparation of Powder formulation

Mixed extract of Coptis Rhizome and *Schizonepeta tenuifolia* Briquet in Example 7-5 500 mg
Lactose 100 mg
Talc 10 mg

The above ingredients were mixed, and a sealed package was filled with the mixture, thereby preparing a powder formulation.

### Formulation Example 2: Preparation of Tablet formulation

Mixed extract of Coptis Rhizome and *Schizonepeta tenuifolia* Briquet in Example 7-5 300 mg
Corn starch 100 mg
Lactose 100 mg
Magnesium stearate 2 mg

The above ingredients were mixed, and then the mixture was tableted according to an ordinary tablet preparation method, thereby preparing a tablet formulation.

### Formulation Example 3: Preparation of Capsule formulation

Mixed extract of Coptis Rhizome and *Schizonepeta tenuifolia* Briquet in Example 7-5 200 mg
Crystalline cellulose 3 mg
Lactose 14.8 mg
Magnesium stearate 0.2 mg

According to an ordinary capsule preparation method, the above ingredients were mixed, and then a gelatin capsule was filled with the mixture, thereby preparing a capsule formulation.

### Formulation Example 4: Preparation of liquid formulation

Mixed extract of Coptis Rhizome and *Schizonepeta tenuifolia* Briquet in Example 7-5 4 g
Saccharide isomerate 10 g
Mannitol 5 g
Purified water appropriate amount

According to an ordinary liquid formulation preparation method, the respective ingredients were added to and dissolved in purified water, followed by the addition of an appropriate amount of lemon flavor, and then the above ingredients were mixed and purified water was added thereto to make a total volume of 100 mL, with which a brown bottle was then filled, followed by sterilization, thereby preparing a liquid formulation.

### Formulation Example 5: Preparation of health food

Mixed extract of Coptis Rhizome and *Schizonepeta tenuifolia* Briquet in Example 7-5 1,000 mg

| Vitamin mixture | appropriate amount |
|---|---|
| Vitamin A acetate | 70 **µg** |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 **µg** |
| Vitamin C | 10 mg |
| Biotin | 10 **µg** |
| Nicotinamide | 1.7 **mg** |
| Folic acid | 50 **µg** |
| Calcium pantothenate | 0.5 mg |
| Mineral mixture | appropriate amount |
| Ferrous sulfate | 1.75 mg |
| Zinc oxide | 0.82 **mg** |
| Magnesium carbonate | 25.3 **mg** |
| Potassium dihydrogen phosphate | 15 mg |
| Calcium monohydrogen phosphate | 55 mg |
| Potassium citrate | 90 **mg** |
| Calcium carbonate | 100 **mg** |
| Magnesium chloride | 24.8 **mg** |

The vitamin and mineral mixtures were prepared by mixing and composing ingredients comparatively suitable for a health food as preferable examples, but the mixing ratio therefor may be optionally changed. According to an ordinary health food manufacturing method, the above ingredients were mixed and granulated, and according to an ordinary method, the granules were used to prepare a health food composition.

### Formulation Example 6: Preparation of health drink

Mixed extract of Coptis Rhizome and *Schizonepeta tenuifolia* Briquet in Example 7-5 1,000 mg

| | |
|---|---|
| Citric acid | 1,000 **mg** |
| Oligosaccharides | 100 g |
| Plum concentrate | 2 g |
| Taurine | 1 g |

Purified water being added to a total of 900 **Mℓ**

According to an ordinary health drink manufacturing method, the above ingredients were mixed, heated with stirring at 85°C for 1 hour, and then the solution thus obtained was filtered, sealed and sterilized in a sterile 2 L vessel, and kept refrigerated. Thereafter, the resulting solution was used to prepare a health drink composition of the present disclosure.

The composition ratio was prepared by mixing ingredients relatively appropriate for favorite drink according to a preferred example, but the mixing ratio of the ingredients may be optionally modified depending on geographic and ethnic preferences, such as target customers, target country, and purposes of use.

## Claims

1. A pharmaceutical composition for preventing or treating inflammatory bowel disease, comprising a mixed extract of Coptis Rhizome and *Schizonepeta tenuifolia* Briquet as an active ingredient.

2. The pharmaceutical composition of claim 1, wherein the inflammatory bowel disease is selected from the group consisting of ulcerative colitis, Crohn's disease, intestinal Behcet's disease, indeterminate colitis, bacterial enteritis, viral enteritis, amoebic enteritis, hemorrhagic rectal ulcer, ischemic colitis, and tuberculous enteritis.

3. The pharmaceutical composition of claim 1, wherein the pharmaceutical composition inhibits the activation of TNF-α, IL-1β, IL-17, IL-23, CCL2, or MIP-2, which is an inflammatory factor.

4. The pharmaceutical composition of claim 1, wherein the mixed extract of Coptis Rhizome and *Schizonepeta tenuifolia* Briquet is a mixed extract in which Coptis Rhizome and *Schizonepeta tenuifolia* Briquet are mixed at a weight ratio of 1-30:1-30.

5. The pharmaceutical composition of claim 1, wherein the mixed extract of Coptis Rhizome and *Schizonepeta tenuifolia* Briquet is obtained by extraction with at least one solvent selected from water, an alcohol having 1 to 4 carbon atoms, or a mixture thereof.

6. The pharmaceutical composition of claim 5, wherein the alcohol is ethanol.

7. The pharmaceutical composition of claim 6, wherein the concentration of the alcohol is 20 to 99%.

8. A food composition for preventing or alleviating inflammatory bowel disease, comprising a mixed extract of Coptis Rhizome and *Schizonepeta tenuifolia* Briquet.

9. A feed composition for preventing or alleviating inflammatory bowel disease, comprising a mixed extract of Coptis Rhizome and *Schizonepeta tenuifolia* Briquet.


<110>    Helixmith Co., Ltd

<120>    PHARMACEUTICAL COMPOSITION COMPRISING A MIXTURE EXTRACT OF COPTIS
         RHIZOME AND SCHIZONEPETA TENUIFOLIA BRIQUET AS AN ACTIVE
         INGREDIENT FOR PREVENTING OR TREATING INFLAMMATORY BOWEL DISEASE

<130>    PP200026

<150>    KR 10-2019-0050088
<151>    2019-04-29

<160>    14

<170>    KoPatentIn 3.0

<210>    1
<211>    19
<212>    DNA
<213>    Artificial Sequence

<220>
<223>    GAPDH forward primer


<400>    1
agcctcgtcc cgtagacaa                                                    19


<210>    2
<211>    20
<212>    DNA
<213>    Artificial Sequence

<220>
<223>    GAPDH Reverse primer


<400>    2
aatctccact ttgccactgc                                                   20


<210>    3
<211>    21
<212>    DNA
<213>    Artificial Sequence

<220>
<223>    TNF-alpha forward primer


<400>    3
gcctcttctc attcctgctt g                                                 21


<210>    4
<211>    20
<212>    DNA
<213>    Artificial Sequence

<220>
<223>    TNF-alpha Reverse primer

<400>    4
ctgatgagag ggaggccatt                                                    20


<210>    5
<211>    21
<212>    DNA
<213>    Artificial Sequence

<220>
<223>    IL-1 beta forward primer


<400>    5
tgtgcaagtg tctgaagcag c                                                  21


<210>    6
<211>    20
<212>    DNA
<213>    Artificial Sequence

<220>
<223>    IL-1 beta reverse primer


<400>    6
tggaagcagc ccttcatctt                                                    20


<210>    7
<211>    20
<212>    DNA
<213>    Artificial Sequence

<220>
<223>    IL-23 forward primer


<400>    7
gctgtgccta ggagtagcag                                                    20


<210>    8
<211>    20
<212>    DNA
<213>    Artificial Sequence

<220>
<223>    IL-23 reverse primer


<400>    8
tggctgttgt ccttgagtcc                                                    20


<210>    9
<211>    23
<212>    DNA
<213>    Artificial Sequence

<220>
<223>    CCL2 forward primer

<400>    9
tcccactcac ctgctgctac tca                                              23


<210>    10
<211>    21
<212>    DNA
<213>    Artificial Sequence

<220>
<223>    CCL2 reverse primer


<400>    10
gcttctttgg gacacctgct g                                                21


<210>    11
<211>    20
<212>    DNA
<213>    Artificial Sequence

<220>
<223>    MIP-2 forward primer


<400>    11
ccctgccaag ggttgacttc                                                  20


<210>    12
<211>    20
<212>    DNA
<213>    Artificial Sequence

<220>
<223>    MIP-2 reverse primer


<400>    12
gcaaactttt tgaccgccct                                                  20


<210>    13
<211>    20
<212>    DNA
<213>    Artificial Sequence

<220>
<223>    IL-17 forward primer


<400>    13
tctccaccgc aatgaagacc                                                  20


<210>    14
<211>    20
<212>    DNA
<213>    Artificial Sequence

<220>
<223>    IL-17 reverse primer

<400>    14
cacacccacc agcatcttct                                                    20